# EUROPEAN PATENT APPLICATION

(11) **EP 4 560 645 A1**
(43) Date of publication of application: **28.05.2025**
(21) Application number: 24210300.0
(22) Date of filing: 31.10.2024
(51) Int. Cl.: G16H 30/40, G16H 50/70, G16H 40/40

(54) **SYSTEM AND METHODS FOR MONITORING TRAINED MACHINE LEARNING MODELS IN A HEALTHCARE CONTEXT**

(30) Priority: 22.11.2023 US 202318518315
(71) Applicant: GE Precision Healthcare LLC, Waukesha, WI 53188 (US)
(72) Inventor: NARAYANAN, Aruna, San Ramon, 94583 (US); ALADAHALLI, Chandan Kumar Mallappa, 560066 Bengaluru (IN); AVINASH, Gopal B., San Ramon, 94583 (US); PURANIK, Hemant R., San Ramon, 94583 (US); BHATIA, Ravi Narain, San Ramon, 94583 (US); BONDALAKUNTA, Sunil Gowtham, 560066 Bengaluru (IN)
(74) Representative: Kilburn & Strode LLP

(57) **Abstract**

The present disclosure provides a system and method (200) for monitoring the performance of Artificial Intelligence (AI) models and applications in the medical imaging domain, without requiring access to the underlying clinical data. The system extracts characteristics of input data (202), model performance (204), and user feedback (206) at the point of encounter and uses these characteristics as a surrogate for the underlying input data. The invention further includes methods (500, 800, 1100) for determining deviations by comparing the extracted characteristics against previously determined characteristics and responding to deviations exceeding a threshold by transmitting an alert to a user device (140).

## Description

### FIELD

Embodiments of the present invention generally relate to the field of artificial intelligence (AI) and machine learning, and more particularly to a system and method for monitoring the performance of AI models and applications in the medical imaging domain.

### BACKGROUND

With the increasing adoption of AI in healthcare, the need to monitor the performance of AI models in real-world scenarios is becoming a mandate. However, due to regulatory restrictions and privacy concerns, access to actual clinical data, such as medical images and personally identifiable information (PHI), is often restricted. This poses a significant challenge for data scientists and AI developers who need to understand how their models are performing in the field and make necessary adjustments to improve their performance and reliability.

Traditionally, AI model performance monitoring in healthcare contexts has utilized the patient clinical data upon which the AI model bases its inference. However, this approach may require obtaining patient consent, overcoming region-specific restrictions, and managing data lifecycle. This process is not only time-consuming and complex but may also raise privacy and security concerns. Therefore, there is a need for a solution that enables monitoring of AI model performance without using PHI or other underlying clinically sensitive data.

### BRIEF DESCRIPTION

In one embodiment, a method for monitoring performance of a trained machine learning model includes, extracting characteristics of input data fed into the trained machine learning model, to produce an input data characterization, wherein the input data includes a medical image, extracting characteristics of performance of the trained machine learning model during mapping of the medical image to an output, to produce a model performance characterization, extracting characteristics of user feedback received based on the output of the trained machine learning model, to produce a user feedback characterization, determining an input data deviation by comparing the input data characterization against a plurality of previously determined input data characterizations, determining a model performance deviation by comparing the model performance characterization against a plurality of previously determined model performance characterizations, determining a user feedback deviation by comparing the user feedback characterization against a plurality of previously determined user feedback characterizations, and responding to one or more of the input data deviation exceeding an input data deviation threshold, the model performance deviation exceeding a model performance deviation threshold, and the user feedback deviation exceeding a user feedback deviation threshold, by transmitting an alert to a user device.

In another embodiment, a system comprising, a first device located at a deployment site, wherein the first device comprises, a user input device, a first non-transitory memory including a trained machine learning model, and instructions, and a first processor, wherein, when executing the instructions, the first processor causes the first device to extract characteristics of input data fed into the trained machine learning model, to produce an input data characterization, wherein the input data includes a medical image, extract characteristics of performance of the trained machine learning model during mapping of the medical image to an output, to produce a model performance characterization, extract characteristics of user feedback received via the user input device based on the output of the trained machine learning model, to produce a user feedback characterization, and transmit the input data characterization, the model performance characterization, and the user feedback characterization, to a second device, the second device located remotely from the first device, wherein the first device and the second device are communicatively coupled, and wherein the second device comprises a second non-transitory memory including instructions, and a second processor, wherein, when executing the instructions, the second processor causes the second device to, receive the input data characterization, the model performance characterization, and the user feedback characterization, from the first device, determine an input data deviation by comparing the input data characterization against a plurality of previously determined input data characterizations, determine a model performance deviation by comparing the model performance characterization against a plurality of previously determined model performance characterizations, determine a user feedback deviation by comparing the user feedback characterization against a plurality of previously determined user feedback characterizations, and respond to one or more of the input data deviation exceeding an input data deviation threshold, the model performance deviation exceeding a model performance deviation threshold, and the user feedback deviation exceeding a user feedback deviation threshold by transmitting an alert to a user device.

In yet another embodiment, a method for monitoring performance of a trained machine learning model comprises, responding to a medical image of an imaging subject being input into the trained machine learning model by, extracting characteristics of the medical image of the imaging subject to produce an input data characterization, wherein the input data characterization excludes individual pixel or voxel intensity values and individual pixel or voxel locations, extracting characteristics of performance of the trained machine learning model during mapping of the medical image to an output, to produce a model performance characterization, determining an input data deviation by comparing the input data characterization against a plurality of previously determined input data characterizations, determining a model performance deviation by comparing the model performance characterization against a plurality of previously determined model performance characterizations, and responding to one or more of the input data deviation exceeding an input data deviation threshold, and the model performance deviation exceeding a model performance deviation threshold by transmitting an alert to a user device.

In this way, the present disclosure enables monitoring of deployed model performance without using PHI or other underlying clinically sensitive data by extracting characteristics of input data, model performance, and user feedback, and using these characteristics as a surrogate for the underlying clinical data. The extracted characteristics may be used to detect deviations in deployed model performance relative to a baseline model performance (e.g., model performance on a test dataset, a validation dataset, and a training dataset), thus, allowing data scientists and developers to gain real-time insights into the performance of deployed models directly accessing underlying clinical data.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

FIG. 1 is a block diagram illustrating an image processing device and a model monitoring device, according to an embodiment of the current disclosure;
FIG. 2 is a flowchart illustrating a method for monitoring performance of a trained machine learning model, according to an embodiment of the current disclosure;
FIG. 3 is a flowchart illustrating a method for characterizing a medical image, according to an embodiment of the current disclosure;
FIG. 4 is a flowchart illustrating a method for characterizing a three-dimensional (3D) medical image, according to an embodiment of the current disclosure;
FIG. 5 is a flowchart illustrating a method for determining a deviation of an input data characterization using a plurality of pre-determined input data characterizations, according to an embodiment of the current disclosure;
FIG. 6 is a flowchart illustrating a method for characterizing output of a trained machine learning model, according to an embodiment of the current disclosure;
FIG. 7 is a flowchart illustrating a method for characterizing a segmentation mask produced by a trained machine learning model, according to an embodiment of the current disclosure;
FIG. 8 is a flowchart illustrating a method for determining a deviation of the output of the trained machine learning model using a plurality of pre-determined model output characterizations, according to an embodiment of the current disclosure;
FIG. 9 is a flowchart illustrating a method for characterizing user feedback for a model output, according to an embodiment of the current disclosure;
FIG. 10 is a flowchart illustrating a method for characterizing user feedback by determining user adjustments to an output of the trained machine learning model, according to an embodiment of the current disclosure;
FIG. 11 is a flowchart illustrating a method for determining a deviation of the user feedback using a plurality of pre-determined user feedback characterizations, according to an embodiment of the current disclosure;
FIG. 12 is a flowchart illustrating a method for characterizing input data from a training dataset used to train the machine learning model, according to an embodiment of the current disclosure;
FIG. 13 is a flowchart illustrating a method for extracting model performance characteristics during training of a machine learning model, according to an embodiment of the current disclosure;
FIG. 14 is a flowchart illustrating a method for extracting ground truth characteristics from a training dataset, according to an embodiment of the current disclosure; and
FIG. 15 is a flowchart illustrating a method for accumulating characterizations from a deployment site, according to an embodiment of the current disclosure.

### DETAILED DESCRIPTION

The present disclosure describes systems and methods for monitoring the performance of trained machine learning models in the medical imaging domain. With the increasing adoption of AI in healthcare, there is a need to monitor the real-world performance of AI models without compromising patient privacy or overcoming regulatory restrictions. The present systems and methods enable performance monitoring by extracting characteristics of the input data, model outputs, and user feedback at the point of clinical use. These characteristics act as surrogates for the underlying clinical data, providing insights into model performance without requiring access to protected health information or clinically sensitive data. The extracted characteristics are compared to expected baselines derived from training data or past model inferences to detect deviations in deployed model performance. Alerts are generated when deviations exceed predetermined thresholds, allowing for timely investigation and intervention. By using statistical surrogates rather than actual clinical data, the present disclosure facilitates continuous performance monitoring to ensure patient safety and model reliability, while maintaining regulatory compliance and protecting sensitive personal information. The systems and methods provide solutions for AI quality assurance in real-world healthcare settings.

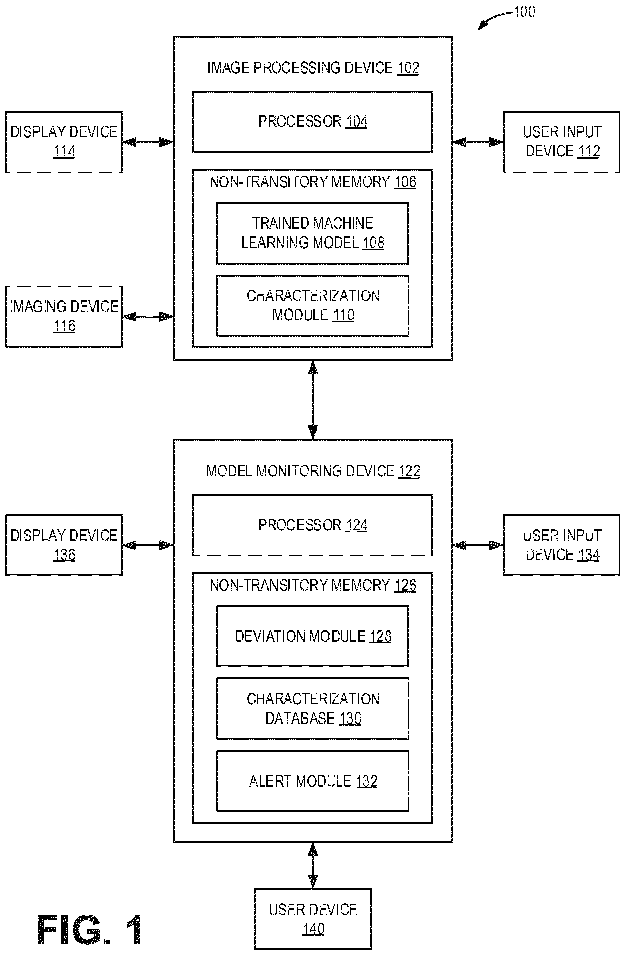

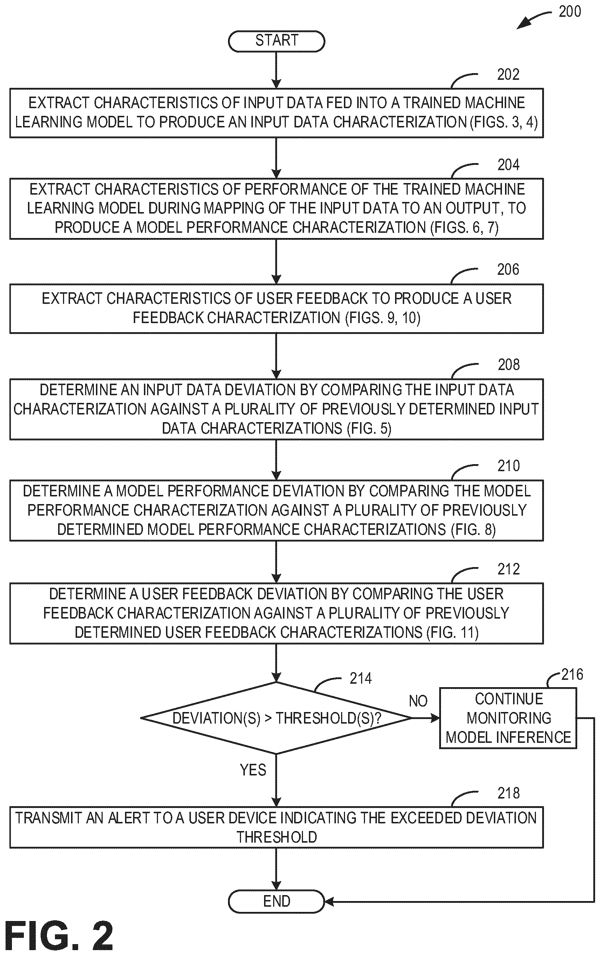

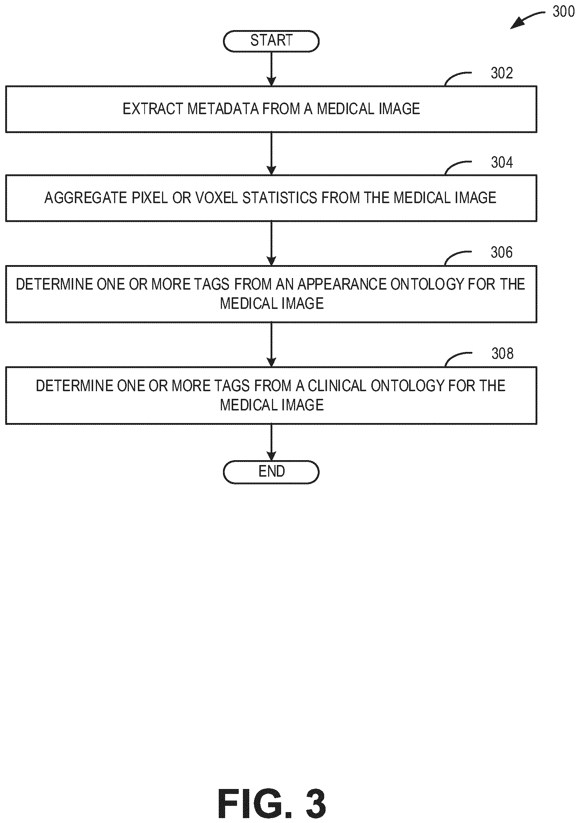

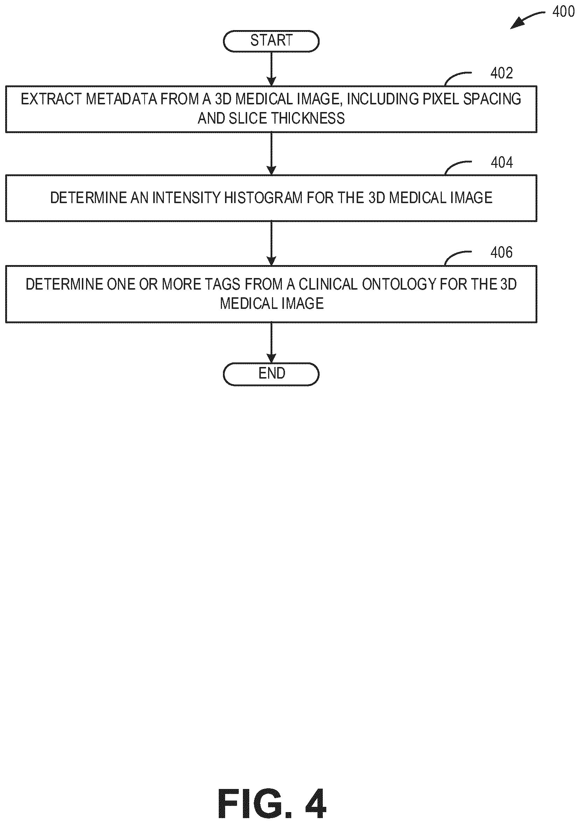

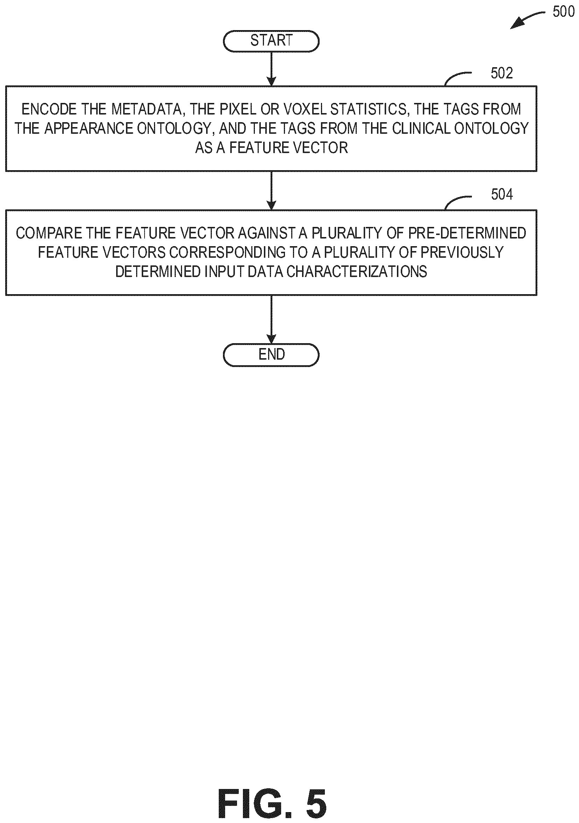

In one example, an image processing device 102 (shown in FIG. 1) located at a deployment site may monitor performance of one or more trained machine learning models by extracting characteristics from input medical image data, model performance, and user feedback. The image processing device 102 transmits the extracted characteristics to a remote model monitoring device 122 that encodes the characteristics as one or more feature vectors, and compares the feature vectors with previously determined characterization vectors to identify deviations in the input data, the model performance, or the user feedback. The system shown in FIG. 1 may execute one or more operations of FIG. 2 to extract characteristic, determine deviations by comparison to previously determined characterizations, and transmit alerts when deviations exceed one or more pre-determined thresholds. Medical image inputs may be characterized using method 300 (shown in FIG. 3) by extracting metadata, pixel statistics, and ontology tags. Additionally, 3D medical image inputs may be characterized using method 400, shown in FIG. 4, wherein metadata, intensity histograms, and clinical ontology tags are used to characterize 3D images. The input data characteristics extracted by methods 300 and/or 400 may be encoded as a vector and compared to previously determined input data characterizations, as shown by method 500 in FIG. 5.

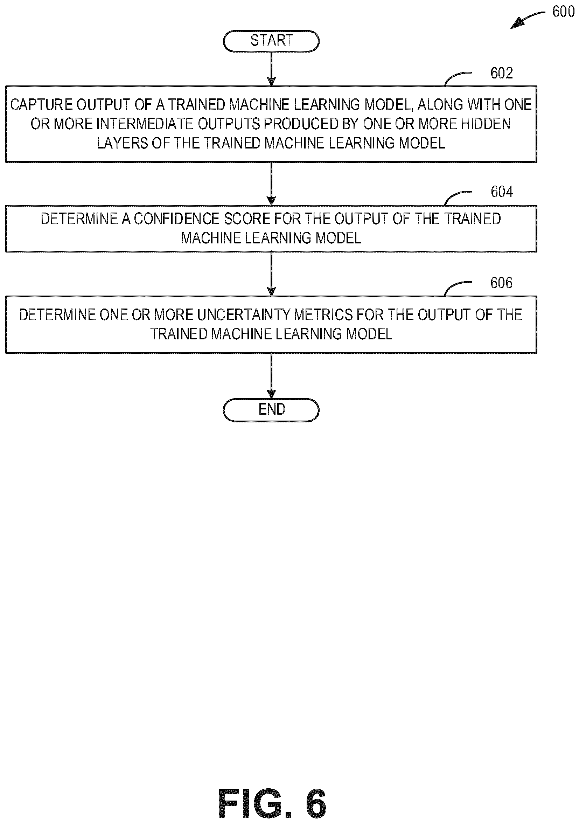

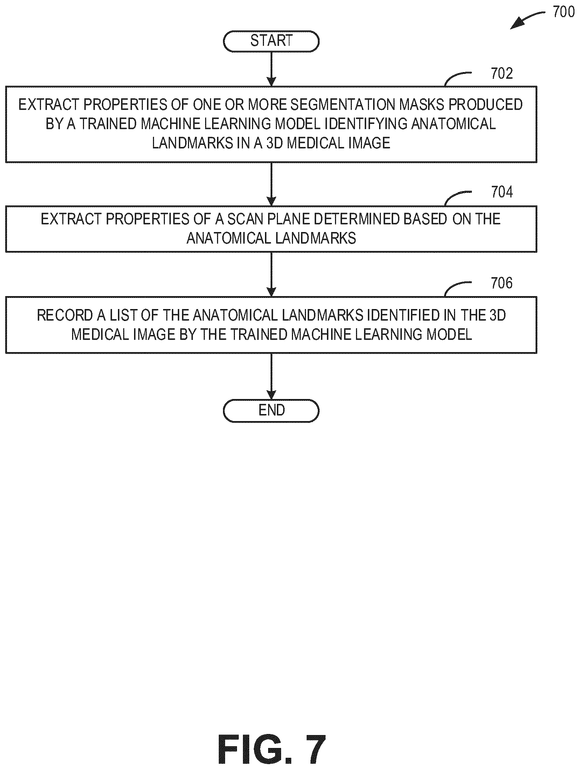

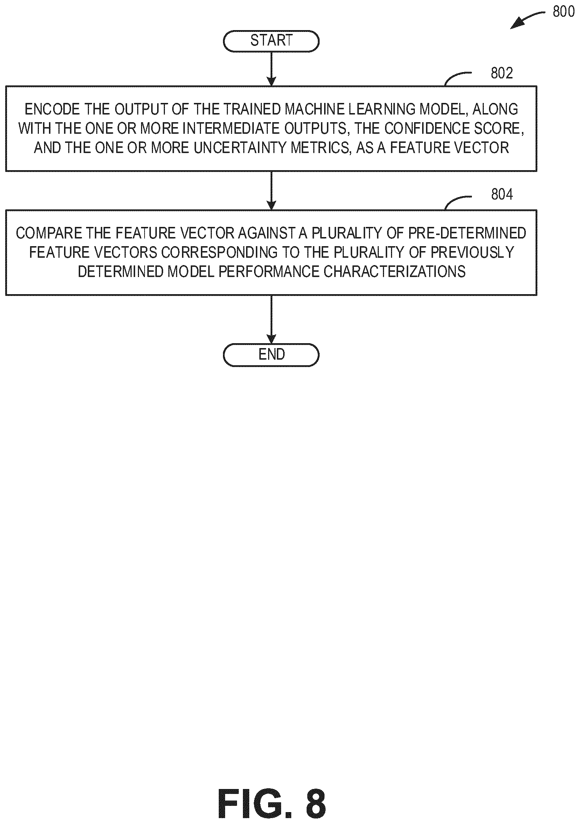

Similarly, model performance may be characterized according to one or more operations of method 600, shown in FIG. 6, including extracting model confidence scores, uncertainty metrics, and other model performance characteristics. In embodiments where model output comprises a segmentation mask, one or more operations of method 700, shown in FIG. 7 may be employed to extract model performance characteristics, such as analyzing properties of the segmentation mask and/or identified landmarks. FIG. 8 shows method 800 for encoding the model performance characteristics produced by methods 600 and or 700 into a feature vector and comparing to previously determined model performance characterization vectors to identify model performance deviations.

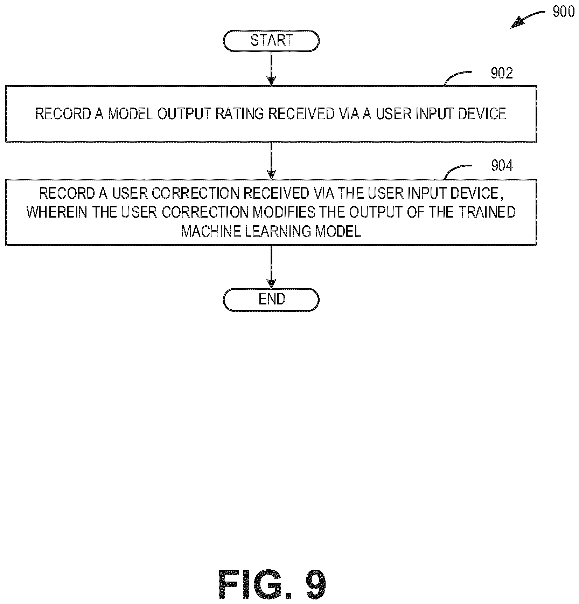

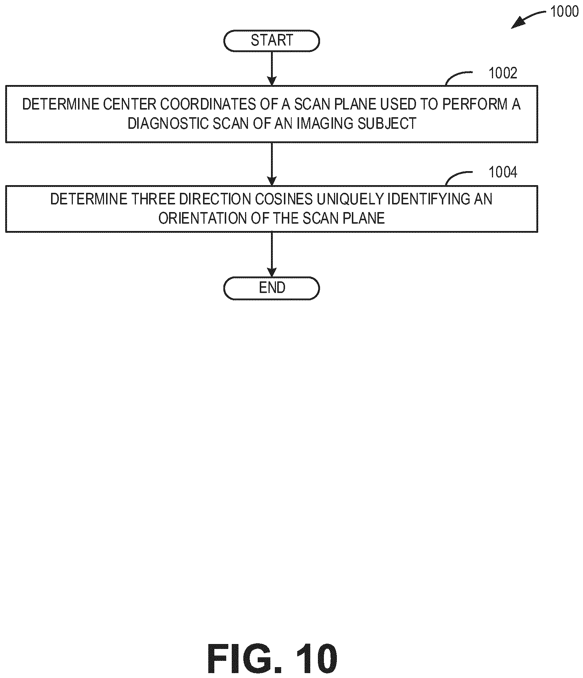

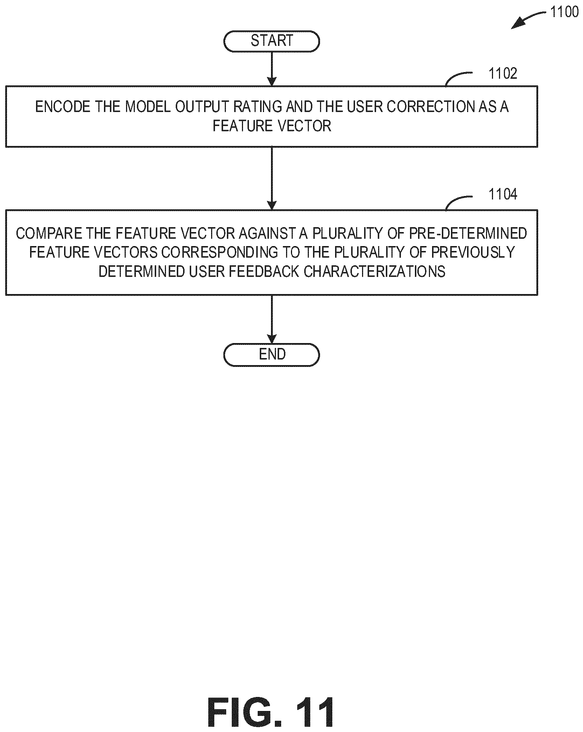

User feedback may be characterized by executing one or more operations of method 900, shown in FIG. 9, which includes capturing user feedback such as model ratings and user provided corrections. In embodiments where model output comprises a scan plane prescription, user feedback may be characterized according to one or more operations of method 1000, shown in FIG. 10, which includes recording scan plane parameters adjusted by the user. The user feedback characteristics determined by methods 900 and/or 1000 may be encoded as a feature vector and compared to previously determined user feedback characterizations to detect deviations by executing one or more operations of method 1100, shown in FIG. 11.

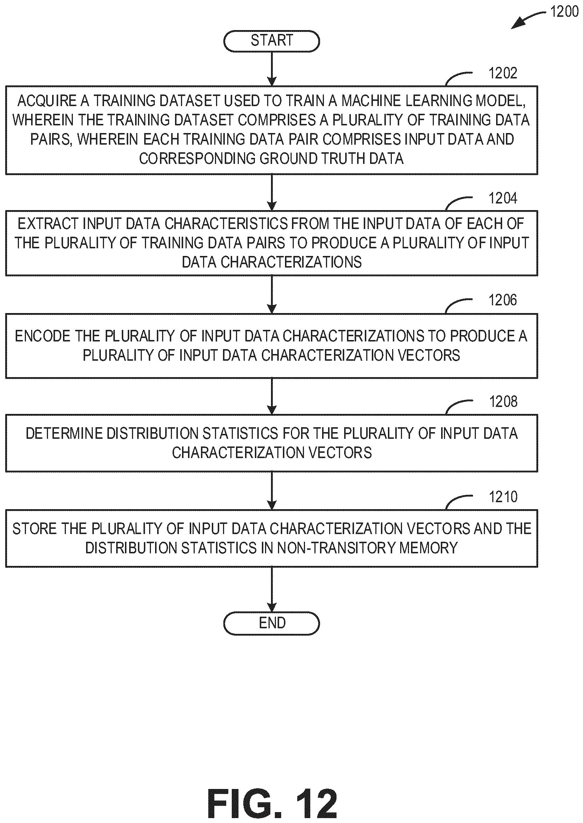

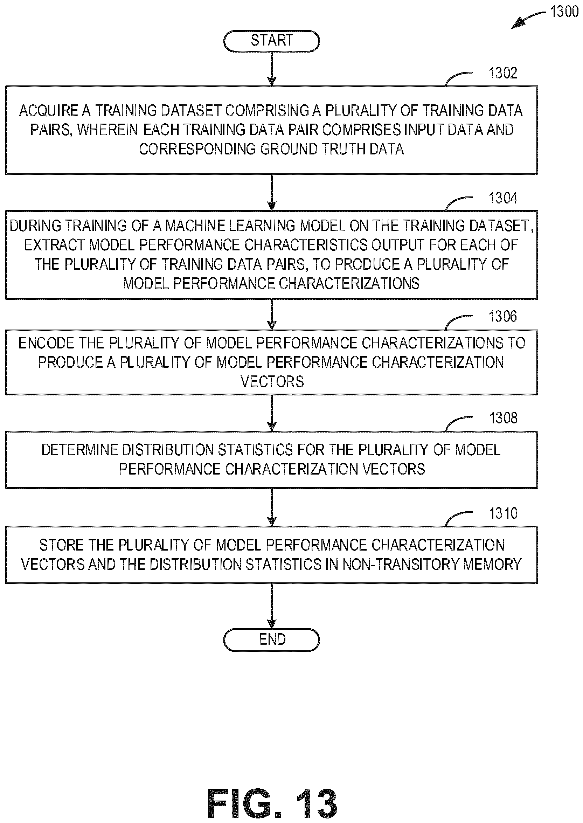

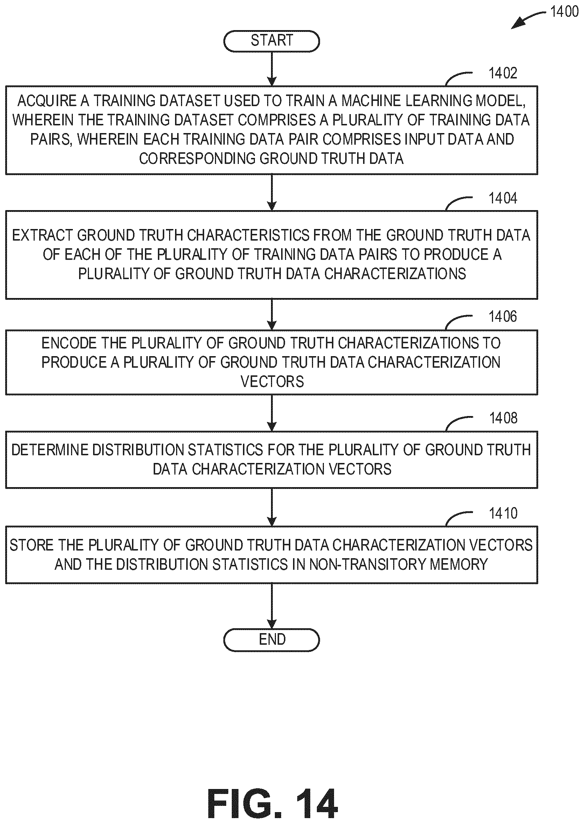

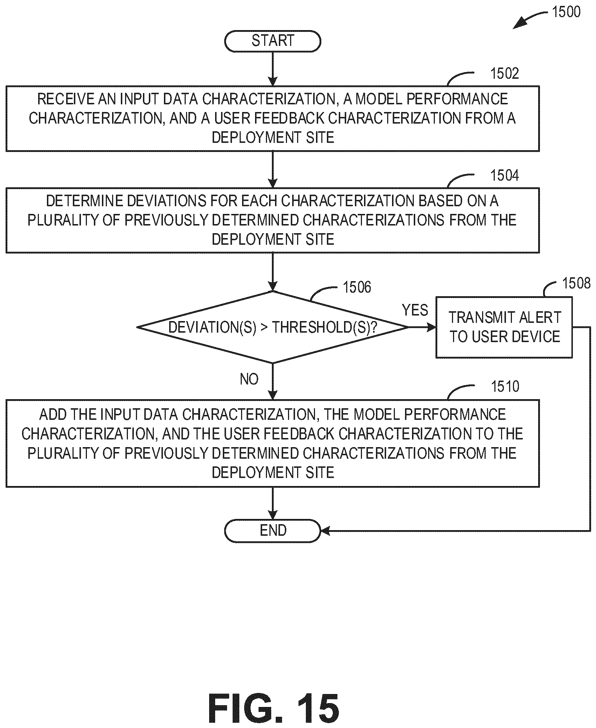

FIG. 12 shows method 1200 for extracting input data characteristics from training data to establish expected/baseline model performance. FIG. 13 details method 1300 for extracting model performance characteristics during training. FIG. 14 outlines techniques in method 1400 for extracting ground truth characteristics from training data. Finally, FIG. 15 illustrates method 1500 for accumulating deployment data characterizations to update performance baselines specific to a deployment site over time. In this way performance and usage of trained machine learning models at a deployment site may be monitored in substantially real time, using statistical surrogates of the underlying clinical data.

Referring first to FIG. 1, a model monitoring system 100 is shown. Model monitoring system 100 may be configured to acquire medical images of an imaging subject using an imaging device 116, and process the acquired medical image using an image processing device 102, e.g., to extract characteristics of the medical image, performance of a trained machine learning model 108, and user feedback received via user input device 112. The processed information may be displayed to a user via display device 114. User input may be received by the image processing device 102 via user input device 112, wherein one or more of image acquisition and image processing may be adjusted based on the user input received. In some embodiments, user input device 112 may be used to provide user feedback to the image processing device 102, such as corrections to the output of the trained machine learning model 108, ratings of the output, and other types of feedback.

Image processing device 102 includes a processor 104 configured to execute machine readable instructions stored in non-transitory memory 106. Processor 104 may be single core or multi-core, and the programs executed thereon may be configured for parallel or distributed processing. In some embodiments, processor 104 may optionally include individual components that are distributed throughout two or more devices, which may be remotely located and/or configured for coordinated processing. In some embodiments, one or more aspects of processor 104 may be virtualized and executed by remotely-accessible networked computing devices configured in a cloud computing configuration.

Non-transitory memory 106 may store the trained machine learning model 108 and the characterization module 110. The trained machine learning model 108 may include instructions for mapping a medical image to an output. The characterization module 110 may include instructions for extracting characteristics of the input data fed into the trained machine learning model 108, characteristics of performance of the trained machine learning model 108 during mapping of the medical image to an output, and characteristics of user feedback received via the user input device 112 based on the output of the trained machine learning model 108. The trained machine learning model 108, stored in the non-transitory memory 106, includes instructions that enable the mapping of a medical image to an output. This output may be a diagnosis, a prediction, or other medically relevant information that may be derived from the medical image. The trained machine learning model 108 may be employ various machine learning algorithms, such as deep learning, convolutional neural networks, or support vector machines, among others. The characterization module 110, also stored in the non-transitory memory 106, includes instructions for extracting various characteristics related to the input data, the performance of the trained machine learning model 108, and the user feedback. The input data characteristics may include metadata from the medical image, pixel or voxel statistics from the medical image, and one or more tags from an appearance ontology and a clinical ontology for the medical image. The metadata does not include personally identifiable information, and the pixel or voxel statistics do not preserve individual pixel or voxel intensity values or locations. The appearance ontology and clinical ontology provide additional context about the medical image, such as the type of pathology and the organs present in the image. The performance characteristics of the trained machine learning model 108 may include the output of the model 108, one or more intermediate outputs produced by one or more hidden layers of the model 108, a confidence score for the output of the model 108, and one or more uncertainty metrics for the output of the model 108. These performance characteristics provide information about how well the model 108 is performing and can be used to identify any potential issues or areas for improvement. The user feedback characteristics may include a model output rating received via a user input device 112 and a user correction received via the user input device 112, wherein the user correction modifies the output of the trained machine learning model 108. This feedback provides insight into how the user interacts with the output of the model 108 and can be used to further refine and improve the model 108.

The image processing device 102 may transmit the input data characterization, the model performance characterization, and the user feedback characterization, to a model monitoring device 122. The model monitoring device 122 is located remotely from the image processing device 102, wherein the image processing device 102 and the model monitoring device 122 are communicatively coupled.

Model monitoring device 122 includes a processor 124 configured to execute machine readable instructions stored in non-transitory memory 126. Processor 124 may be single core or multi-core, and the programs executed thereon may be configured for parallel or distributed processing. In some embodiments, processor 124 may optionally include individual components that are distributed throughout two or more devices, which may be remotely located and/or configured for coordinated processing. In some embodiments, one or more aspects of processor 124 may be virtualized and executed by remotely-accessible networked computing devices configured in a cloud computing configuration.

Non-transitory memory 126 may store the deviation module 128, the characterization database 130, and the alert module 132. The deviation module 128 may include instructions for determining an input data deviation by comparing the input data characterization against a plurality of previously determined input data characterizations, determining a model performance deviation by comparing the model performance characterization against a plurality of previously determined model performance characterizations, and determining a user feedback deviation by comparing the user feedback characterization against a plurality of previously determined user feedback characterizations.

The characterization database 130 may store the plurality of previously determined input data characterizations, model performance characterizations, and user feedback characterizations. These characterizations may be derived from a training dataset used to train the machine learning model or from previous inferences of the trained machine learning model at the deployment site. The characterization database 130 may also store distribution statistics associated with the plurality of input data characterizations, model performance characterizations, and user feedback characterizations. These distribution statistics can be used to dynamically adjust the respective deviation thresholds based on the distribution of the previously determined characterizations. The characterization database 130 may further store a plurality of predetermined feature vectors corresponding to the plurality of previously determined input data characterizations, model performance characterizations, and user feedback characterizations. These pre-determined feature vectors can be used to determine how similar the current characterizations are to the previously determined characterizations, by calculating a distance between the current feature vector and each of the pre-determined feature vectors in the multi-dimensional space. The characterization database 130 may be updated continuously as new characterizations are received from the deployment site, allowing the system to adapt its monitoring to changes in the input data, the performance of the model, or the user feedback.

The alert module 132 may include instructions for responding to one or more of the input data deviation exceeding an input data deviation threshold, the model performance deviation exceeding a model performance deviation threshold, and the user feedback deviation exceeding a user feedback deviation threshold by transmitting an alert to a user device 140.

User input device 134 may comprise one or more of a touchscreen, a keyboard, a mouse, a trackpad, a motion sensing camera, or other device configured to enable a user to interact with and manipulate data within model monitoring device 122.

Display device 136 may include one or more display devices utilizing virtually any type of technology. The display device 136 may be configured to visually present information to a user. This information may include, for example, the input data characterization, the model performance characterization, and the user feedback characterization. The display device 136 may also present the determined deviations of these characterizations from their respective previously determined characterizations. In the event that one or more of the deviations exceed their respective thresholds, the display device 136 may present an alert generated by the alert module 132. This alert may include an indication of which deviation exceeded its threshold, such as the input data deviation exceeding the input data deviation threshold, the model performance deviation exceeding the model performance deviation threshold, or the user feedback deviation exceeding the user feedback deviation threshold. The display device 136 may present this alert in a variety of formats, such as a pop-up notification, a highlighted text, or an audible alarm, depending on the user's preferences and the urgency of the situation. In addition to presenting alerts, the display device 136 may also display various other types of information related to the operation of the trained machine learning model. Display device 136 may be combined with processor 124, non-transitory memory 126, and/or user input device 134 in a shared enclosure, or may be peripheral display devices and may comprise a monitor, touchscreen, projector, or other display device known in the art, which may enable a user to view MRIs produced by an MRI system, and/or interact with various data stored in non-transitory memory 126.

It should be understood that the image processing device 102 and model monitoring device 122 shown in FIG. 1 are for illustration, not for limitation. Another appropriate imaging system and model monitoring device may include more, fewer, or different components.

Referring to FIG. 2, a method 200 for monitoring the performance of a trained machine learning model is shown. The method 200 may be employed by an image processing system to extract characteristics of input data, model performance, and user feedback, and to determine deviations by comparing these characteristics against previously determined characteristics.

At operation 202, the image processing system extracts characteristics of input data fed into the trained machine learning model to produce an input data characterization. This extraction allows the system to identify specific features within the input data that are of interest for monitoring. In one embodiment, the extraction of the input data characteristics involves utilizing metadata from the medical image, such as subject-related characteristics (e.g., age range, sex, BMI), machine-related characteristics (e.g., machine model name), acquisition-related characteristics (e.g., field strength, coil, scan type, orientation, number of slices), and clinical data characteristics (e.g., mean intensity, median intensity, intensity histogram). The metadata does not include personally identifiable information, and the pixel or voxel statistics do not preserve individual pixel or voxel intensity values or locations. The appearance ontology and clinical ontology provide additional context about the medical image, such as the type of pathology and the organs present in the image, without revealing protected health information (PHI).

At operation 204, the image processing system extracts characteristics of performance of the trained machine learning model during mapping of the input data to an output, to produce a model performance characterization. This extraction allows the system to monitor the performance of the machine learning model. In one embodiment, the extraction of the model performance characteristics involves capturing the output of the trained machine learning model, along with one or more intermediate outputs produced by one or more hidden layers of the trained machine learning model, determining a confidence score for the output of the trained machine learning model, and determining one or more uncertainty metrics for the output of the trained machine learning model. In another embodiment, at operation 204, the image processing system may capture model performance characteristics such as classification, segmentation, image regression, point regression, line regression, and plane regression metrics.

At operation 206, the image processing system extracts characteristics of user feedback to produce a user feedback characterization. This extraction allows the system to monitor the user's interaction with the output of the machine learning model. In one embodiment, the extraction of the user feedback characteristics involves recording a model output rating received via a user input device, recording a user correction received via the user input device, wherein the user correction modifies the output of the trained machine learning model, and capturing additional user comments or actions that indicate the user's acceptance or modification of the model's predictions. This feedback may be used to assess the model's alignment with user expectations and clinical workflow requirements.

At operation 208, the image processing system determines an input data deviation by comparing the input data characterization against a plurality of previously determined input data characterizations. This determination allows the system to monitor changes in the input data over time, such as variations in subject demographics, equipment settings, and acquisition parameters, which may impact the model's performance.

At operation 210, the image processing system determines a model performance deviation by comparing the model performance characterization against a plurality of previously determined model performance characterizations. This determination allows the system to monitor changes in the performance of the machine learning model over time, including deviations in classification accuracy, segmentation quality, and regression predictions, which may signal the need for model retraining or adjustment.

At operation 212, the image processing system determines a user feedback deviation by comparing the user feedback characterization against a plurality of previously determined user feedback characterizations. This determination allows the system to monitor changes in the user's interaction with the output of the machine learning model over time, providing insights into the model's utility and acceptance in the clinical environment.

At operation 214, the image processing system evaluates if one or more of the input data deviation, the model performance deviation, and the user feedback deviation exceed their respective thresholds. If any of the deviations exceed their respective thresholds, the image processing system proceeds to operation 218, where it transmits an alert to a user device indicating the exceeded deviation threshold. The alert may include details of the deviation and suggestions for corrective actions. If none of the deviations exceed their respective thresholds, the image processing system continues monitoring the model inference at operation 216. Following operation 218 or operation 216, method 200 may end.

Thus, method 200 may at least partially address the technical challenge of monitoring and maintaining the performance of machine learning models in the medical imaging domain while adhering to strict privacy regulations and without direct access to protected health information (PHI). The technical improvements provided by method 200 include the ability to extract surrogate data characterizations from input data, model performance, and user feedback, which serve as non-invasive proxies for the underlying clinical data. This approach enables the real-time detection of deviations in model performance by comparing these characterizations against a baseline established from previously determined characterizations. When deviations exceed predetermined thresholds, the system generates alerts, facilitating prompt corrective actions to ensure the model's reliability and accuracy. This technical effect ensures that the machine learning models continue to function optimally within the healthcare environment, providing dependable support for medical professionals and enhancing patient care outcomes.

Referring to FIG. 3, a method 300 for characterizing a medical image is shown. The method 300 may be employed by an image processing system to extract characteristics of the medical image, which can be used to monitor the performance of a trained machine learning model.

At operation 302, the image processing system extracts metadata from the medical image. The metadata may include information about the medical image that does not include personally identifiable information (PII) or protected health information (PHI). For example, the metadata may include information about the subject of the image, such as whether the subject is a pediatric subject, the laterality protocol, the pixel spacing, slice thickness, and so on. In some embodiments, the metadata may include information about the subject of the image, such as age range, sex, and body mass index (BMI) computed from weight, height, and sex. Additionally, the metadata may include machine-related characteristics such as the machine model name of the equipment producing the medical image. This metadata provides a high-level overview of the medical image and can be used to characterize the input data fed into the trained machine learning model.

At operation 304, the image processing system aggregates pixel or voxel statistics from the medical image. These statistics do not preserve individual pixel or voxel intensity values or locations but instead provide a summary of the overall intensity distribution within the image. For example, the image processing system may generate an intensity histogram, intensity profiles across anatomical planes such as anteroposterior (AP), superoinferior (SI), and laterorotary (LR), and other aggregate statistics such as mean intensity, median intensity, minimum intensity, and maximum intensity. These statistics can provide insights into the overall quality and characteristics of the medical image, which can be useful for monitoring the performance of the trained machine learning model.

At operation 306, the image processing system determines one or more tags from an appearance ontology for the medical image. The appearance ontology may include a set of predefined tags that describe various visual features or characteristics of the medical image. For example, the tags may describe the type of pathology present in the image, the organs visible in the image, and other visual features such as the presence of artifacts like shadows or blurring. These tags provide a way to categorize and describe the visual content of the medical image in a structured and standardized way.

At operation 308, the image processing system determines one or more tags from a clinical ontology for the medical image. The clinical ontology may include a set of predefined tags that describe various clinical features or characteristics of the medical image. For example, the tags may describe the type of medical imaging modality used to capture the image (e.g., MR, CT, ultrasound), the type of clinical task associated with the image (e.g., classification, segmentation, image regression), and other clinical features such as the presence of specific organs within the field of view (FoV). These tags provide a way to categorize and describe the clinical content of the medical image in a structured and standardized way.

Following operation 308, method 300 may end. The extracted metadata, pixel or voxel statistics, and ontology tags collectively form an input data characterization for the medical image. This input data characterization can be used to monitor the performance of a trained machine learning model by comparing it against previously determined input data characterizations.

Referring to FIG. 4, a method 400 for characterizing a three-dimensional (3D) medical image is shown. The method 400 may be employed by an image processing system to extract characteristics of the 3D medical image, which can be used to produce an input data characterization. This characterization can be used to monitor the performance of a trained machine learning model, particularly in the context of medical imaging.

At operation 402, the image processing system extracts metadata from the 3D medical image. This metadata may include information about the image that does not directly represent the image content, such as pixel spacing and slice thickness. Pixel spacing refers to the physical distance in the patient between the centers of each pixel, measured in millimeters. Slice thickness refers to the thickness of the 3D slice represented by the image, also measured in millimeters. This metadata can provide important context for understanding the image and can be used to normalize or standardize the image data for further processing.

At operation 404, the image processing system determines an intensity histogram for the 3D medical image. An intensity histogram is a graphical representation of the distribution of pixel intensities in an image. In a 3D medical image, the pixel intensities can represent different tissue types or structures, and the intensity histogram can provide a summary of the distribution of these different tissues or structures in the image. The intensity histogram can be used to identify features or patterns in the image data that may be relevant for the trained machine learning model.

At operation 406, the image processing system determines one or more tags from a clinical ontology for the 3D medical image. A clinical ontology is a structured set of terms or tags that represent concepts in the clinical domain. These tags can be used to annotate or describe the content of the 3D medical image in a standardized way. For example, the tags may indicate what organs or anatomical structures are visible in the image, what type of imaging modality was used to acquire the image, or what type of pathology is present in the image. These tags can provide a high-level summary of the image content that can be used to categorize or classify the image for further processing.

Following operation 406, method 400 may end. The extracted metadata, intensity histogram, and clinical ontology tags collectively form an input data characterization for the 3D medical image. This characterization can be used to monitor the performance of a trained machine learning model by comparing it against previously determined input data characterizations.

Referring to FIG. 5, a method 500 for encoding the metadata, pixel or voxel statistics, tags from the appearance ontology, and tags from the clinical ontology as a feature vector, and comparing the feature vector against a plurality of pre-determined feature vectors corresponding to a plurality of previously determined input data characterizations is shown. The method 500 may be employed by an image processing system to determine deviations in the input data characterization, which can be used to monitor the performance of a trained machine learning model.

Referring to FIG. 5, a method 500 for encoding the metadata, pixel or voxel statistics, tags from the appearance ontology, and tags from the clinical ontology as a feature vector, and comparing the feature vector against a plurality of pre-determined feature vectors corresponding to a plurality of previously determined input data characterizations is shown. The method 500 may be employed by an image processing system to determine deviations in the input data characterization, which can be used to monitor the performance of a trained machine learning model.

At operation 502, the image processing system encodes the metadata, pixel or voxel statistics, tags from the appearance ontology, and tags from the clinical ontology as a feature vector. This encoding allows the system to represent the input data characterization in a form that can be easily compared with other input data characterizations. In one embodiment, the encoding involves transforming the metadata, pixel or voxel statistics, and tags into a numerical format that can be represented as a vector in a multi-dimensional space. The dimensions of this space may correspond to the different types of characteristics that are being encoded, such as the metadata, pixel or voxel statistics, and tags. The encoding may also involve normalizing the values of the characteristics to ensure that they are on a similar scale, which can help to prevent any one characteristic from dominating the comparison due to its scale. The encoding may be performed using various techniques, such as one-hot encoding for categorical data, scaling for numerical data, and so on. Additionally, the encoding may leverage specific healthcare-related metadata, such as DICOM metadata, and may include pixel or voxel characteristics derived from the medical images, such as mean intensity, histogram, or entropy, without including any patient-identifiable information, thereby ensuring de-identification of the data.

At operation 504, the image processing system compares the feature vector against a plurality of pre-determined feature vectors corresponding to a plurality of previously determined input data characterizations. This comparison allows the system to determine how similar the current input data characterization is to the previously determined input data characterizations. In one embodiment, the comparison involves calculating a distance between the feature vector and each of the pre-determined feature vectors in the multi-dimensional space. The distance may be calculated using various distance measures, such as Euclidean distance, Manhattan distance, cosine similarity, and so on. The system may then determine a deviation by comparing the calculated distance against a threshold. If the distance exceeds the threshold, this may indicate that the current input data characterization is significantly different from the previously determined input data characterizations, which may in turn indicate a potential issue with the performance of the trained machine learning model. The system may respond to such a deviation by transmitting an alert to a user device, as described in the method 200 shown in FIG. 2. Following operation 504, method 500 may end.

At operation 504, the image processing system compares the feature vector against a plurality of pre-determined feature vectors corresponding to a plurality of previously determined input data characterizations. This comparison allows the system to determine how similar the current input data characterization is to the previously determined input data characterizations. In one embodiment, the comparison involves calculating a distance between the feature vector and each of the pre-determined feature vectors in the multi-dimensional space. The distance may be calculated using various distance measures, such as Euclidean distance, Manhattan distance, cosine similarity, and so on. The system may then determine a deviation by comparing the calculated distance against a threshold. If the distance exceeds the threshold, this may indicate that the current input data characterization is significantly different from the previously determined input data characterizations, which may in turn indicate a potential issue with the performance of the trained machine learning model. The system may respond to such a deviation by transmitting an alert to a user device, as described in the method 200 shown in FIG. 2. The alert may be generated by an alert module 132 and transmitted to a user device 140, which may be part of a hospital or data scientist's monitoring setup. Following operation 504, method 500 may end.

Referring to FIG. 6, a method 600 for characterizing output of a trained machine learning model in the context of medical imaging is shown. The method 600 may be employed by an image processing system to capture the output of the trained machine learning model, determine a confidence score for the output, and determine one or more uncertainty metrics for the output, which may collectively characterize performance of the trained machine learning model in mapping the input data to a desired output.

At operation 602, the image processing system captures the output of the trained machine learning model, along with one or more intermediate outputs produced by one or more hidden layers of the trained machine learning model. This operation allows the system to monitor the performance of the model at various stages of its operation. The output of the model may include, for example, the identification of anatomical landmarks in a 3D medical image, the properties of a scan plane determined based on the anatomical landmarks, and a list of the anatomical landmarks identified in the 3D medical image by the trained machine learning model. The intermediate outputs may include, for example, the results of individual layers or groups of layers in the model, which can provide insight into the internal workings of the model and help identify potential areas of improvement or correction.

At operation 604, the image processing system determines a confidence score for the output of the trained machine learning model. The confidence score provides a quantitative measure of the model's certainty or reliability in its output. The confidence score may be determined based on various factors, such as the model's internal parameters, the quality of the input data, and the complexity of the task at hand. For example, the confidence score may be higher if the input data is of high quality and the task is relatively simple, and lower if the input data is of low quality or the task is complex. The confidence score can be used to assess the performance of the model and to decide whether to trust its output or to seek additional information or confirmation.

At operation 606, the image processing system determines one or more uncertainty metrics for the output of the trained machine learning model. The uncertainty metrics provide a quantitative measure of the model's uncertainty or variability in its output. The uncertainty metrics may be determined based on various factors, such as the model's internal parameters, the quality of the input data, and the complexity of the task at hand. For example, the uncertainty metrics may be higher if the input data is of low quality or the task is complex, and lower if the input data is of high quality and the task is relatively simple. The uncertainty metrics can be used to assess the performance of the model and to decide whether to trust its output or to seek additional information or confirmation.

Following operation 606, method 600 may end. This method allows for the continuous monitoring and evaluation of the performance of a trained machine learning model in the context of medical imaging, which can help ensure the accuracy and reliability of the model's output, and ultimately improve the quality of patient care.

Referring to FIG. 7, a method 700 for producing a model output characterization by determining properties of one or more segmentation masks produced by a trained machine learning model and identifying anatomical landmarks in a 3D medical image, is shown. The method 700 may be employed by an image processing system to extract properties of segmentation masks, which are used to identify anatomical landmarks in a 3D medical image.

At operation 702, the image processing system extracts properties of one or more segmentation masks produced by a trained machine learning model identifying anatomical landmarks in a 3D medical image. The segmentation masks are generated by the trained machine learning model and are used to identify and isolate specific anatomical landmarks within the 3D medical image. The properties of these segmentation masks may include, but are not limited to, the size of the segmentation mask, the centroid of the segmentation mask, and the intensity profile within the segmentation mask. These properties provide valuable information about the anatomical landmarks and can be used to further characterize the input data for the machine learning model.

In one embodiment, the size of the segmentation mask may be determined by counting the number of pixels or voxels within the mask, which can provide an estimate of the volume or area of the anatomical landmark that the mask represents. The centroid of the segmentation mask, which is the geometric center of the mask, can be calculated by averaging the coordinates of all the pixels or voxels within the mask. The intensity profile within the segmentation mask can be determined by calculating statistics such as the mean, median, standard deviation, and histogram of the pixel or voxel intensities within the mask. Additionally, the segmentation mask properties may include an uncertainty score that quantifies the confidence of the segmentation, and the segmentation mask type, which may be a bounding box with dimensions, a polygon with vertices, or a base-64-encoded mask representing the segmented area.

At operation 704, the image processing system extracts properties of a scan plane determined based on the anatomical landmarks. The scan plane is a two-dimensional slice of the 3D medical image that is determined based on the positions of the anatomical landmarks. The properties of the scan plane may include, but are not limited to, the orientation of the plane, the position of the plane within the 3D medical image, and the pixel or voxel intensities within the plane. These properties can provide additional information about the anatomical landmarks and the surrounding tissue or structures.

In one embodiment, the orientation of the scan plane can be represented by three direction cosines, which are the cosines of the angles that the plane's normal vector makes with the axes of the image coordinate system. The position of the scan plane within the 3D medical image can be represented by the coordinates of the plane's center point. The pixel or voxel intensities within the plane can be characterized by calculating statistics such as the mean, median, standard deviation, and histogram of the intensities. Furthermore, in some embodiments, the scan plane properties may include the extents of the plane, which can be represented by the dimensions in the x, y, and z directions, and the center, which can be represented by the coordinates in the image coordinate system.

At operation 706, the image processing system records a list of the anatomical landmarks identified in the 3D medical image by the trained machine learning model. This list can include the names or labels of the landmarks, their positions within the 3D medical image, and their associated confidence scores, which indicate the model's certainty in the identification of each landmark. This list can be used to further characterize the input data for the machine learning model and to monitor the model's performance. Following operation 706, method 700 may end.

Referring to FIG. 8, a method 800 for determining a deviation of a model performance characterization from a plurality of previously determined model performance characterization, is shown. The method 800 may be employed by a model monitoring system to determine deviations in the performance of the machine learning model based on the characteristics of the model output.

At operation 802, the model monitoring system encodes the output of the trained machine learning model, along with one or more intermediate outputs, the confidence score, and one or more uncertainty metrics, as a feature vector. This encoding allows the system to capture a comprehensive representation of the model's performance. The output of the trained machine learning model may include the final prediction or decision made by the model, such as the identification of anatomical landmarks in a medical image. The intermediate outputs may include the outputs of one or more hidden layers of the model, which provide insight into the internal workings of the model. The confidence score may represent the model's certainty in its output, and the uncertainty metrics may quantify the model's uncertainty or variability in its output.

At operation 802, the model monitoring system encodes the output of the trained machine learning model, along with one or more intermediate outputs, the confidence score, and one or more uncertainty metrics, as a feature vector. This encoding allows the system to capture a comprehensive representation of the model's performance. The output of the trained machine learning model may include the final prediction or decision made by the model, such as the identification of anatomical landmarks in a medical image, classification of medical images into predetermined categories, segmentation of specific structures within medical images, or regression analysis to predict continuous variables from medical images. The intermediate outputs may include the outputs of one or more hidden layers of the model, which provide insight into the internal workings of the model. The confidence score may represent the model's certainty in its output, and the uncertainty metrics may quantify the model's uncertainty or variability in its output, including uncertainty scores associated with predicted masks, points, lines, or planes in medical images.

At operation 804, the model monitoring system compares the feature vector against a plurality of pre-determined feature vectors corresponding to a plurality of previously determined model performance characterizations. This comparison allows the system to determine how the current performance of the model deviates from its past performance or expected performance. The pre-determined feature vectors may be derived from a training dataset used to train the machine learning model, or from previous inferences of the model at the deployment site. The comparison may involve calculating a distance or similarity measure between the feature vector and each of the pre-determined feature vectors, such as the Euclidean distance, cosine similarity, or other suitable distance or similarity measures. If the comparison reveals that the deviation of the model's performance exceeds a model performance deviation threshold, the model monitoring system may respond by transmitting an alert to a user device. The alert may indicate that the model's performance has deviated significantly from its expected performance, and may suggest potential actions to address the deviation, such as re-training the model, adjusting the model's parameters, or investigating the cause of the deviation. Following operation 804, method 800 may end.

Referring to FIG. 9, a method 900 for characterizing user feedback received based on output from the trained machine learning model, is shown. The method 900 may be employed by an image processing system to extract characteristics of user feedback received based on the output of the trained machine learning model, to produce a user feedback characterization.

At operation 902, the image processing system records a model output rating received via a user input device. This model output rating may be a numerical or categorical value that represents the user's assessment of the quality or accuracy of the output of the trained machine learning model. The model output rating may be based on various factors, such as the perceived accuracy of the output, the usefulness of the output in the user's workflow, the user's confidence in the output, and so on. The model output rating may be input by the user via a user input device, such as a keyboard, a mouse, a touchscreen, a voice command interface, or any other suitable input device.

At operation 904, the image processing system records a user correction received via the user input device, wherein the user correction modifies the output of the trained machine learning model. The user correction may be an adjustment or modification made by the user to the output of the trained machine learning model. The user correction may include, for example, adjusting the position or orientation of a segmentation mask, adding or removing landmarks, adjusting the parameters of a recommended scan plane, and so on. The user correction may be input by the user via the user input device, and may be recorded by the image processing system as part of the user feedback characterization.

The user feedback characterization produced by method 900 provides valuable information about how the trained machine learning model is performing in the real-world context of the user's workflow. By analyzing the user feedback characterization, the image processing system can identify areas where the trained machine learning model may need improvement, and can use this information to update or retrain the model, thereby improving its performance and usefulness in the healthcare context. Following operation 904, method 900 may end.

Referring to FIG. 10, a method 1000 for characterizing user feedback received based on the output of a trained machine learning model is shown. The method 1000 may be employed by an image processing system to determine the user feedback characterization, which may be used in monitoring the performance of Al models and applications in the medical imaging domain.

At operation 1002, the image processing system determines the center coordinates of a scan plane used to perform a diagnostic scan of an imaging subject. This determination allows the system to identify a final, user approved/adjusted scan plane that is used to perform a diagnostic scan.

At operation 1004, the image processing system determines three direction cosines uniquely identifying an orientation of the scan plane. Following operation 1004, method 1000 may end.

The user feedback characterization, which includes the center coordinates of the scan plane and the three direction cosines uniquely identifying an orientation of the scan plane, provides information about a user's interaction with the AI model. This information can be used to monitor the performance of the AI model and to identify any deviations from expected behavior. If such deviations are detected, an alert can be transmitted to a user device, allowing for timely intervention and correction.

Referring to FIG. 11, a method 1100 for determining a deviation of the user feedback using a plurality of pre-determined user feedback characterizations is shown. The method 1100 may be employed by an image processing system to compare the user feedback characterization against a plurality of previously determined user feedback characterizations.

At operation 1102, the image processing system encodes the model output rating and the user correction as a feature vector. The model output rating and the user correction are received via a user input device and are part of the user feedback characterization. The model output rating may be a numerical or categorical value indicating the user's assessment of the quality or accuracy of the output of the trained machine learning model. The user correction may be a modification made by the user to the output of the trained machine learning model, such as an adjustment to the position or orientation of a scan plane, or a change to the identified landmarks or segmentation masks. The encoding of the model output rating and the user correction as a feature vector allows for a compact and efficient representation of the user feedback characterization, which can be easily compared against other feature vectors representing previously determined user feedback characterizations.

At operation 1104, the image processing system compares the feature vector against a plurality of pre-determined feature vectors corresponding to the plurality of previously determined user feedback characterizations. This comparison allows the image processing system to determine a user feedback deviation, which is a measure of the difference between the current user feedback characterization and the previously determined user feedback characterizations. The user feedback deviation may be calculated as a distance in the feature space between the feature vector and the closest one of the pre-determined feature vectors, or as a statistical measure of the difference between the feature vector and a distribution of the pre-determined feature vectors. The user feedback deviation provides an indication of how much the current user feedback deviates from the typical or expected user feedback, which can be used to monitor the performance of the trained machine learning model and to detect any anomalies or issues in its operation.

In some embodiments, the image processing system may respond to the user feedback deviation exceeding a user feedback deviation threshold by transmitting an alert to a user device. The user feedback deviation threshold may be a pre-determined value or a value that is dynamically adjusted based on the distribution of the previously determined user feedback characterizations. The alert may include information about the user feedback deviation and suggestions for corrective actions, such as re-training the machine learning model, adjusting its parameters, or providing additional guidance to the user. Following operation 1104, method 1100 may end.

Referring to FIG. 12, a method 1200 for characterizing input data from a training dataset used to train a machine learning model is shown. The method 1200 may be employed by an image processing system to extract input data characteristics from the input data of each of the plurality of training data pairs to produce a plurality of input data characterizations, which may subsequently be used as a baseline of comparison for determining deviations of input data characterizations for input data received at a deployment site.

At operation 1202, the image processing system acquires a training dataset used to train a machine learning model. The training dataset comprises a plurality of training data pairs, wherein each training data pair comprises input data and corresponding ground truth data. The input data may include medical images, such as MR, CT, or ultrasound images, and the ground truth data may include annotations or labels that indicate the presence or absence of certain features or conditions in the images.

At operation 1204, the image processing system extracts input data characteristics from the input data of each of the plurality of training data pairs to produce a plurality of input data characterizations. This extraction process may involve one or more of the operations described in more detail above with respect to methods 300 and 400. The characteristics may include metadata, pixel or voxel statistics, and tags from appearance and clinical ontologies. The metadata may include information about the imaging subject, such as whether the subject is a pediatric subject, the laterality protocol, the pixel spacing, slice thickness, and so on. The pixel or voxel statistics may include intensity histograms, intensity profiles, noise, and other statistical measures derived from the pixel or voxel values in the images. The tags from the appearance and clinical ontologies may include information about the type of pathology, the organs present in the images, and other high-level characteristics that cannot be easily derived from the pixel data alone.

At operation 1206, the image processing system encodes the plurality of input data characterizations to produce a plurality of input data characterization vectors. This encoding process may involve converting the extracted characteristics into a numerical form that can be easily compared and analyzed. For example, the metadata, pixel or voxel statistics, and ontology tags may be encoded as a feature vector, where each element of the vector corresponds to a particular characteristic.

At operation 1208, the image processing system determines distribution statistics for the plurality of input data characterization vectors. These statistics may include measures of central tendency, such as the mean or median, measures of dispersion, such as the variance or standard deviation, and other statistical measures that describe the distribution of the input data characterization vectors. These statistics provide a summary of the characteristics of the input data in the training dataset, and can be used to compare the input data characteristics of new data against the characteristics of the training data.

At operation 1210, the image processing system stores the plurality of input data characterization vectors and the distribution statistics in non-transitory memory. This stored information can be used later to compare the characteristics of new input data against the characteristics of the training data, in order to monitor the performance of the trained machine learning model and detect any deviations or drifts in the input data. Following operation 1210, method 1200 may end.

Referring to FIG. 13, a method 1300 for extracting model performance characteristics during the training of a machine learning model is shown. The method 1300 may be employed by an image processing system to extract model performance characteristics output for each of the plurality of training data pairs, to produce a plurality of model performance characterizations.

At operation 1302, the image processing system acquires a training dataset used to train a machine learning model. The training dataset comprises a plurality of training data pairs, wherein each training data pair comprises input data and corresponding ground truth data. The input data may include medical images, such as MR or CT scans, ultrasound images, or other types of medical imaging data. The ground truth data may include annotations or labels that indicate the presence or absence of certain features or conditions in the input data, such as the presence of a particular pathology, the location of certain anatomical landmarks, or the correct classification of the input data.

At operation 1304, during the training of the machine learning model on the training dataset, the image processing system extracts model performance characteristics output for each of the plurality of training data pairs. This extraction process may involve capturing the output of the trained machine learning model, along with one or more intermediate outputs produced by one or more hidden layers of the trained machine learning model. The model performance characteristics may include, for example, the confidence score for the output of the trained machine learning model, and one or more uncertainty metrics for the output of the trained machine learning model. The confidence score may indicate the degree of certainty with which the model makes its predictions, while the uncertainty metrics may provide a measure of the model's uncertainty or variability in its predictions.

At operation 1306, the image processing system encodes the plurality of model performance characterizations to produce a plurality of model performance characterization vectors. This encoding process may involve transforming the model performance characteristics into a format that can be easily compared and analyzed. For example, the model performance characteristics may be encoded as a feature vector, where each element of the vector corresponds to a different model performance characteristic.

At operation 1308, the image processing system determines distribution statistics for the plurality of model performance characterization vectors. These distribution statistics may include measures of central tendency, such as the mean or median, and measures of dispersion, such as the variance or standard deviation. The distribution statistics provide a summary of the overall performance of the machine learning model during its training phase.

At operation 1310, the image processing system stores the plurality of model performance characterization vectors and the distribution statistics in non-transitory memory. This stored information can be used later to monitor the performance of the machine learning model when it is deployed in a real-world setting, by comparing the model's performance on new data against the performance characteristics that were observed during training. Following operation 1310, method 1300 may end.

Referring to FIG. 14, a method 1400 for extracting ground truth characteristics from a training dataset used to train a machine learning model is shown. The method 1400 may be employed by an image processing system to extract ground truth characteristics from the ground truth data of each of the plurality of training data pairs to produce a plurality of ground truth data characterizations.

At operation 1402, the image processing system acquires a training dataset used to train a machine learning model. The training dataset comprises a plurality of training data pairs, wherein each training data pair comprises input data and corresponding ground truth data. The ground truth data may include information such as the correct classification or output for the corresponding input data. This ground truth data serves as a reference for the machine learning model during the training process, enabling the model to learn the correct mapping from the input data to the desired output.

At operation 1404, the image processing system extracts ground truth characteristics from the ground truth data of each of the plurality of training data pairs to produce a plurality of ground truth data characterizations. This extraction process may involve various techniques depending on the nature of the ground truth data. For instance, if the ground truth data includes labels for image classification, the ground truth characteristics may include the distribution of labels in the dataset. If the ground truth data includes bounding boxes for object detection, the ground truth characteristics may include the size, shape, and location of the bounding boxes. If the ground truth data includes segmentation masks for image segmentation, the ground truth characteristics may include the area, perimeter, and centroid of the segmentation masks.

At operation 1406, the image processing system encodes the plurality of ground truth characterizations to produce a plurality of ground truth data characterization vectors. This encoding process may involve transforming the ground truth characteristics into a numerical format that can be easily processed. For instance, the ground truth characteristics may be normalized, scaled, or otherwise transformed to produce the ground truth data characterization vectors.

At operation 1408, the image processing system determines distribution statistics for the plurality of ground truth data characterization vectors. These distribution statistics may include measures such as the mean, median, mode, variance, standard deviation, skewness, kurtosis, and other statistical measures of the ground truth data characterization vectors. These distribution statistics provide a summary of the ground truth data characterization vectors, enabling the image processing system to understand the overall characteristics of the ground truth data in the training dataset.

At operation 1410, the image processing system stores the plurality of ground truth data characterization vectors and the distribution statistics in non-transitory memory. This stored information can be used later for various purposes, such as monitoring the performance of the trained machine learning model, detecting drift in the input data, or adjusting the training process of the machine learning model. Following operation 1410, method 1400 may end.

Referring to FIG. 15, a method 1500 for accumulating input data characterizations, model performance characterizations, and user feedback characterizations, from a particular deployment site, is shown. The method 1500 may be employed by a model monitoring system establish a baseline for various model performance metrics for a particular deployment site.

At operation 1502, the model monitoring system receives an input data characterization, a model performance characterization, and a user feedback characterization from a deployment site. The input data characterization may include characteristics of input data fed into the trained machine learning model, such as metadata from a medical image, pixel or voxel statistics from the medical image, and one or more tags from an appearance ontology and a clinical ontology for the medical image. The model performance characterization may include characteristics of performance of the trained machine learning model during mapping of the medical image to an output, such as the output of the trained machine learning model, one or more intermediate outputs produced by one or more hidden layers of the trained machine learning model, a confidence score for the output of the trained machine learning model, and one or more uncertainty metrics for the output of the trained machine learning model. The user feedback characterization may include characteristics of user feedback received based on the output of the trained machine learning model, such as a model output rating and a user correction that modifies the output of the trained machine learning model.

At operation 1504, the model monitoring system determines deviations for each characterization based on a plurality of previously determined characterizations from the deployment site. This determination involves comparing the received characterizations against the previously determined characterizations to identify any deviations. The previously determined characterizations may be derived from a training dataset used to train the trained machine learning model, or from previous inferences of the trained machine learning model at the deployment site.

At operation 1506, the model monitoring system checks if any of the determined deviations exceed a respective threshold. If none of the deviations exceed their respective threshold, the model monitoring system proceeds to operation 1510, wherein the received characterizations are added to the plurality of previously determined characterizations from the deployment site. This allows the model monitoring system to continuously update its understanding of the "normal" operation of the trained machine learning model, and to adapt its monitoring to changes in the input data, the performance of the model, or the user feedback.

If at operation 1506, one or more of the deviations exceed their respective thresholds, the model monitoring system proceeds to operation 1508, wherein an alert is transmitted to a user device indicating the exceeded deviation threshold. The alert may include information about the nature of the deviation, the magnitude of the deviation, and potential corrective actions. The alert may be transmitted via various communication channels, such as email, text message, push notification, or other suitable communication methods. Following operations 1510 or 1508, method 1500 may end.

The above disclosed system and methods for monitoring performance of trained machine learning models in a healthcare context enable an improvement in the computational efficiency of real time monitoring of deployed model performance. By extracting surrogate data characterizations from input data, model performance, and user feedback, the system eliminates the need to process and analyze the full volume of sensitive medical images and associated PHI. This approach reduces the computational load on the system, as the characterizations are lightweight compared to the original data and can be processed rapidly to detect performance deviations. The system's ability to encode these characterizations as feature vectors further enhances computational efficiency, allowing for quick comparisons against baselines. This streamlined process not only conserves computational resources but also enables real-time monitoring and rapid response to deviations, ensuring that the machine learning models operate within pre-determined performance parameters, e.g., within a pre-determined deviation from performance achieved during model training. The computational efficiency of the above disclosed system and methods may be particularly advantageous in healthcare settings, where timely and accurate model performance is paramount, and computational resources may be limited.

The disclosure also provides support for a method comprising: extracting characteristics of input data fed into a trained machine learning model, to produce an input data characterization, wherein the input data includes a medical image, extracting characteristics of performance of the trained machine learning model during mapping of the medical image to an output, to produce a model performance characterization, extracting characteristics of user feedback received based on the output of the trained machine learning model, to produce a user feedback characterization, determining an input data deviation by comparing the input data characterization against a plurality of previously determined input data characterizations, determining a model performance deviation by comparing the model performance characterization against a plurality of previously determined model performance characterizations, determining a user feedback deviation by comparing the user feedback characterization against a plurality of previously determined user feedback characterizations, and responding to one or more of the input data deviation exceeding an input data deviation threshold, the model performance deviation exceeding a model performance deviation threshold, and the user feedback deviation exceeding a user feedback deviation threshold, by transmitting an alert to a user device. In a first example of the method, extracting characteristics of input data fed into the trained machine learning model to produce the input data characterization comprises: extracting metadata from the medical image, wherein the metadata does not include personally identifiable information, aggregating pixel or voxel statistics from the medical image, wherein the pixel or voxel statistics do not preserve individual pixel or voxel intensity values or locations, determining one or more tags from an appearance ontology for the medical image, and determining one or more tags from a clinical ontology for the medical image. In a second example of the method, optionally including the first example, determining the input data deviation by comparing the input data characterization against the plurality of previously determined input data characterizations comprises: encoding the metadata, the pixel or voxel statistics, the one or more tags from the appearance ontology, and the one or more tags from the clinical ontology, as a feature vector, and comparing the feature vector against a plurality of pre-determined feature vectors corresponding to the plurality of previously determined input data characterizations. In a third example of the method, optionally including one or both of the first and second examples, extracting characteristics of performance of the trained machine learning model during mapping of the medical image to the output, to produce the model performance characterization comprises: capturing the output of the trained machine learning model, along with one or more intermediate outputs produced by one or more hidden layers of the trained machine learning model, determining a confidence score for the output of the trained machine learning model, and determining one or more uncertainty metrics for the output of the trained machine learning model. In a fourth example of the method, optionally including one or more or each of the first through third examples, determining the model performance deviation by comparing the model performance characterization against the plurality of previously determined model performance characterizations comprises: encoding the output of the trained machine learning model, along with the one or more intermediate outputs, the confidence score, and the one or more uncertainty metrics, as a feature vector, and comparing the feature vector against a plurality of pre-determined feature vectors corresponding to the plurality of previously determined model performance characterizations. In a fifth example of the method, optionally including one or more or each of the first through fourth examples, extracting characteristics of user feedback received based on the output of the trained machine learning model, to produce the user feedback characterization comprises: recording one or more of: a model output rating received via a user input device, and a user correction received via the user input device, wherein the user correction modifies the output of the trained machine learning model. In a sixth example of the method, optionally including one or more or each of the first through fifth examples, extracting characteristics of user feedback received based on the output of the trained machine learning model, to produce the user feedback characterization further comprises: receiving comments from a user via the user input device, and determining a sentiment score for the output of the trained machine learning model based on the comments. In a seventh example of the method, optionally including one or more or each of the first through sixth examples, determining the user feedback deviation by comparing the user feedback characterization against the plurality of previously determined user feedback characterizations comprises: encoding the model output rating and the user correction as a feature vector, and comparing the feature vector against a plurality of predetermined feature vectors corresponding to the plurality of previously determined user feedback characterizations.

The disclosure also provides support for a system comprising: a first device located at a deployment site, wherein the first device comprises: a user input device, a first non-transitory memory including a trained machine learning model, and instructions, and a first processor, wherein, when executing the instructions, the first processor causes the first device to: extract characteristics of input data fed into the trained machine learning model, to produce an input data characterization, wherein the input data includes a medical image, extract characteristics of performance of the trained machine learning model during mapping of the medical image to an output, to produce a model performance characterization, extract characteristics of user feedback received via the user input device based on the output of the trained machine learning model, to produce a user feedback characterization, and transmit the input data characterization, the model performance characterization, and the user feedback characterization, to a second device, the second device located remotely from the first device, wherein the first device and the second device are communicatively coupled, and wherein the second device comprises: a second non-transitory memory including instructions, and a second processor, wherein, when executing the instructions, the second processor causes the second device to: receive the input data characterization, the model performance characterization, and the user feedback characterization, from the first device, determine an input data deviation by comparing the input data characterization against a plurality of previously determined input data characterizations, determine a model performance deviation by comparing the model performance characterization against a plurality of previously determined model performance characterizations, determine a user feedback deviation by comparing the user feedback characterization against a plurality of previously determined user feedback characterizations, and respond to one or more of the input data deviation exceeding an input data deviation threshold, the model performance deviation exceeding a model performance deviation threshold, and the user feedback deviation exceeding a user feedback deviation threshold by transmitting an alert to a user device. In a first example of the system, the plurality of previously determined input data characterizations, the plurality of previously determined model performance characterizations, and the plurality of previously determined user feedback characterizations, are derived from a training dataset used to train the trained machine learning model. In a second example of the system, optionally including the first example, the plurality of previously determined input data characterizations, the plurality of previously determined model performance characterizations, and the plurality of previously determined user feedback characterizations, are derived from previous inferences of the trained machine learning model at the deployment site. In a third example of the system, optionally including one or both of the first and second examples, the input data characterization, the model performance characterization, and the user feedback characterization, each do not include the medical image, and wherein when executing the instructions, the first processor does not transmit the medical image to the second device. In a fourth example of the system, optionally including one or more or each of the first through third examples, the alert includes an indication of one or more of the input data deviation exceeding the input data deviation threshold, the model performance deviation exceeding the model performance deviation threshold, and the user feedback deviation exceeding the user feedback deviation threshold.

The disclosure also provides support for a method for monitoring performance of a trained machine learning model, comprising: responding to a medical image of an imaging subject being input into the trained machine learning model by: extracting characteristics of the medical image of the imaging subject to produce an input data characterization, wherein the input data characterization excludes individual pixel or voxel intensity values and individual pixel or voxel locations, extracting characteristics of performance of the trained machine learning model during mapping of the medical image to an output, to produce a model performance characterization, determining an input data deviation by comparing the input data characterization against a plurality of previously determined input data characterizations, determining a model performance deviation by comparing the model performance characterization against a plurality of previously determined model performance characterizations, and responding to one or more of the input data deviation exceeding an input data deviation threshold, and the model performance deviation exceeding a model performance deviation threshold by transmitting an alert to a user device. In a first example of the method the method further comprising: extracting characteristics of user feedback received based on the output of the trained machine learning model, to produce a user feedback characterization, and determining a user feedback deviation by comparing the user feedback characterization against a plurality of previously determined user feedback characterizations, and responding to the user feedback deviation exceeding a user feedback deviation threshold by transmitting an alert to the user device. In a second example of the method, optionally including the first example, the user feedback comprises a scan plane used to acquire a diagnostic medical image of the imaging subject. In a third example of the method, optionally including one or both of the first and second examples extracting characteristics of user feedback received based on the output of the trained machine learning model, to produce the user feedback characterization, comprises: determining center coordinates of the scan plane, and determining three direction cosines uniquely identifying an orientation of the scan plane. In a fourth example of the method, optionally including one or more or each of the first through third examples, the medical image comprises a three-dimensional (3D) medical image, and wherein the trained machine learning model is configured to identify anatomical landmarks in the 3D medical image for positioning of a scan plane for acquisition of a diagnostic medical image of a region of interest. In a fifth example of the method, optionally including one or more or each of the first through fourth examples, extracting characteristics of the medical image of the imaging subject to produce the input data characterization comprises: capturing metadata of the 3D medical image, including pixel spacing and slice thickness of the 3D medical image, determining aggregate intensity statistics for the 3D medical image, including an intensity histogram, and determining a clinical ontology for the 3D medical image comprising a list of anatomical regions captured in the 3D medical image. In a sixth example of the method, optionally including one or more or each of the first through fifth examples, extracting characteristics of performance of the trained machine learning model during mapping of the medical image to the output, to produce the model performance characterization comprises: extracting properties of one or more segmentation masks produced by the trained machine learning model identifying the anatomical landmarks in the 3D medical image, extracting properties of the scan plane determined based on the anatomical landmarks, and recording a list of the anatomical landmarks identified in the 3D medical image by the trained machine learning model.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. A method (200) comprising:
extracting characteristics of input data fed into a trained machine learning model (108), to produce an input data characterization (202), wherein the input data includes a medical image;
extracting characteristics of performance of the trained machine learning model (108) during mapping of the medical image to an output, to produce a model performance characterization (204);
extracting characteristics of user feedback received based on the output of the trained machine learning model (108), to produce a user feedback characterization (206);
determining an input data deviation by comparing the input data characterization against a plurality of previously determined input data characterizations (500);
determining a model performance deviation by comparing the model performance characterization against a plurality of previously determined model performance characterizations (800);
determining a user feedback deviation by comparing the user feedback characterization against a plurality of previously determined user feedback characterizations (1100); and
responding to one or more of the input data deviation exceeding an input data deviation threshold, the model performance deviation exceeding a model performance deviation threshold, and the user feedback deviation exceeding a user feedback deviation threshold, by transmitting an alert to a user device (140).

2. The method (200) of claim 1, wherein extracting characteristics of input data fed into the trained machine learning model (108) to produce the input data characterization (202) comprises:
extracting metadata from the medical image, wherein the metadata does not include personally identifiable information (302);
aggregating pixel or voxel statistics from the medical image (304), wherein the pixel or voxel statistics do not preserve individual pixel or voxel intensity values or locations;
determining one or more tags from an appearance ontology for the medical image (306); and
determining one or more tags from a clinical ontology for the medical image (308).

3. The method (200) of claim 2, wherein determining the input data deviation by comparing the input data characterization against the plurality of previously determined input data characterizations (500) comprises:
encoding the metadata, the pixel or voxel statistics, the one or more tags from the appearance ontology, and the one or more tags from the clinical ontology, as a feature vector (502); and
comparing the feature vector against a plurality of pre-determined feature vectors corresponding to the plurality of previously determined input data characterizations (504).

4. The method (200) of claim 1, wherein extracting characteristics of performance of the trained machine learning model (108) during mapping of the medical image to the output, to produce the model performance characterization (204) comprises:
capturing the output of the trained machine learning model (108), along with one or more intermediate outputs produced by one or more hidden layers of the trained machine learning model (108, 602);
determining a confidence score for the output of the trained machine learning model (108, 604); and
determining one or more uncertainty metrics for the output of the trained machine learning model (108, 606).

5. The method (200) of claim 4, wherein determining the model performance deviation by comparing the model performance characterization against the plurality of previously determined model performance characterizations (800) comprises:
encoding the output of the trained machine learning model (108), along with the one or more intermediate outputs, the confidence score, and the one or more uncertainty metrics, as a feature vector (802); and
comparing the feature vector against a plurality of pre-determined feature vectors corresponding to the plurality of previously determined model performance characterizations (804).

6. The method (200) of claim 1, wherein extracting characteristics of user feedback received based on the output of the trained machine learning model (108), to produce the user feedback characterization (206) comprises:
recording one or more of:
a model output rating received via a user input device (112, 134, 902); and
a user correction received via the user input device (112, 134, 904), wherein the user correction modifies the output of the trained machine learning model (108).

7. The method (200) of claim 6, wherein extracting characteristics of user feedback received based on the output of the trained machine learning model (108), to produce the user feedback characterization (206) further comprises:
receiving comments from a user via the user input device (112, 134); and
determining a sentiment score for the output of the trained machine learning model (108) based on the comments.

8. The method (200) of claim 6, wherein determining the user feedback deviation by comparing the user feedback characterization against the plurality of previously determined user feedback characterizations (1100) comprises:
encoding the model output rating and the user correction as a feature vector (1102); and
comparing the feature vector against a plurality of pre-determined feature vectors corresponding to the plurality of previously determined user feedback characterizations (1104).

9. A system (100) comprising:
a first device (102) located at a deployment site, wherein the first device (102) comprises:
a user input device (112);
a first non-transitory memory (106) including a trained machine learning model (108), and instructions; and
a first processor (104), wherein, when executing the instructions, the first processor (104) causes the first device (102) to:
extract characteristics of input data fed into the trained machine learning model (108), to produce an input data characterization, wherein the input data includes a medical image;
extract characteristics of performance of the trained machine learning model (108) during mapping of the medical image to an output, to produce a model performance characterization;
extract characteristics of user feedback received via the user input device (112) based on the output of the trained machine learning model (108), to produce a user feedback characterization; and
transmit the input data characterization, the model performance characterization, and the user feedback characterization, to a second device (122);
the second device (122) located remotely from the first device (102), wherein the first device (102) and the second device (122) are communicatively coupled, and wherein the second device (122) comprises:
a second non-transitory memory (126) including instructions; and
a second processor (124), wherein, when executing the instructions, the second processor (124) causes the second device (122) to:
receive the input data characterization, the model performance characterization, and the user feedback characterization, from the first device (102);
determine an input data deviation by comparing the input data characterization against a plurality of previously determined input data characterizations;
determine a model performance deviation by comparing the model performance characterization against a plurality of previously determined model performance characterizations;
determine a user feedback deviation by comparing the user feedback characterization against a plurality of previously determined user feedback characterizations; and
respond to one or more of the input data deviation exceeding an input data deviation threshold, the model performance deviation exceeding a model performance deviation threshold, and the user feedback deviation exceeding a user feedback deviation threshold by transmitting an alert to a user device (140).

10. The system (100) of claim 9, wherein the plurality of previously determined input data characterizations, the plurality of previously determined model performance characterizations, and the plurality of previously determined user feedback characterizations, are derived from a training dataset used to train the trained machine learning model (108).

11. The system (100) of claim 9, wherein the plurality of previously determined input data characterizations, the plurality of previously determined model performance characterizations, and the plurality of previously determined user feedback characterizations, are derived from previous inferences of the trained machine learning model (108) at the deployment site.

12. The system (100) of claim 9, wherein the input data characterization, the model performance characterization, and the user feedback characterization, each do not include the medical image, and wherein when executing the instructions, the first processor (104) does not transmit the medical image to the second device (122).

13. The system (100) of claim 9, wherein the alert includes an indication of one or more of the input data deviation exceeding the input data deviation threshold, the model performance deviation exceeding the model performance deviation threshold, and the user feedback deviation exceeding the user feedback deviation threshold.

14. The system (100) of claim 9, wherein, when executing the instructions, the first processor (104) is configured to extract characteristics of the input data fed into the trained machine learning model (108), to produce the input data characterization, by:
extracting metadata from the medical image, wherein the metadata does not include personally identifiable information;
aggregating pixel or voxel statistics from the medical image, wherein the pixel or voxel statistics do not preserve individual pixel or voxel intensity values or locations;
determining one or more tags from an appearance ontology for the medical image; and
determining one or more tags from a clinical ontology for the medical image.

15. The system (100) of claim 14, wherein, when executing the instructions, the first processor (104) is configured to determine the input data deviation by comparing the input data characterization against the plurality of previously determined input data characterizations by:
encoding the metadata, the pixel or voxel statistics, the one or more tags from the appearance ontology, and the one or more tags from the clinical ontology, as a feature vector; and
comparing the feature vector against a plurality of pre-determined feature vectors corresponding to the plurality of previously determined input data characterizations.
